# EUROPEAN PATENT SPECIFICATION

(11) **EP 3 525 887 B1**
(45) Date of publication and mention of the grant of the patent: **04.11.2020**
(21) Application number: 17771777.4
(22) Date of filing: 27.09.2017
(51) Int. Cl.: A61Q 5/00, A61K 8/49, A61P 43/00, A61K 31/352

(54) **ANTI-DANDRUFF COMPOSITION**
ANTISCHUPPENZUSAMMENSETZUNG
COMPOSITION ANTIPELLICULAIRE

(30) Priority: 13.10.2016 EP 16193626
(43) Date of publication of application: 21.08.2019
(73) Proprietor: Unilever PLC, London, Greater London EC4Y 0DY (GB); Unilever N.V., 3013 AL Rotterdam (NL)
(72) Inventor: GINGER, Rebecca, Susan, Bedford Bedfordshire MK44 1LQ (GB); POPLE, Jennifer, Elizabeth, Bedford Bedfordshire MK44 1LQ (GB)
(74) Representative: Chisem, Janet
(86) International application number: PCT/EP2017/074471
(87) International publication number: WO 2018/069045

(56) References cited:
- WO-A1-03/096998
- DATABASE GNPD [Online] MINTEL; 1 September 2013 (2013-09-01), ANONY: "ANONYMOUS", XP002777088, Database accession no. 2155595
- JUSTYNA MIERZIAK ET AL: "Flavonoids as Important Molecules of Plant Interactions with the Environment", MOLECULES, vol. 19, no. 10, 10 October 2014 (2014-10-10), pages 16240-16265, XP055348864, DOI: 10.3390/molecules191016240

## Description

### Field of the Invention

The present invention relates, *inter alia*, to an anti-dandruff composition, the novel use of certain anti-dandruff agents, a method of making an anti-dandruff composition, and to a method of treating and/or preventing dandruff.

### Background of the Invention

Hair treatment compositions are well known and have been widely used for long time. Beside the basic function of cleaning, the hair treatment composition can also deliver the benefit of conditioning, anti-dandruff, cooling etc. by including the corresponding functional additive into the composition. However, when some beneficial agents are included into the composition, the interaction between the ingredients may become very complicated.

Dandruff is a common skin condition, the cause or causes of which are not well understood. The current consensus is that dandruff is a multifactorial condition with three major influencing factors: *Malassezia* fungus colonization, sebum production, and individual predisposition. It is known to treat the condition by application of treatment products such as shampoos and/or conditioners comprising anti-dandruff active compounds. Among the best known active anti-dandruff compounds are metal pyrithiones, such as zine pyrithione, and anti-fungals such as ketoconazole and climbazole, and various sulphides of selenium.

However, it is not unknown for anti-dandruff treatments to lose their effectiveness, and certain types of anti-dandruff shampoo are not recommended for use by women who are pregnant or breast-feeding. There is therefore a need for alternative anti-dandruff treatments, especially if based on a safe active agent, such as may be found naturally occurring in plants or plant-derived products. It is however fundamentally challenging to try and identify new, effective approaches to treating dandruff. This is because dandruff is a multifactorial condition involving interaction of factors associated with skin, sebum and skin microflora and currently the interaction of these factors, and the processes leading to the development of dandruff, are not fully understood. In addition, whilst it is known that some parameters differ between healthy skin and dandruff, it is not clear if these differences represent cause or effect. JUSTYNA MIERZIAK ET AL: "Flavonoids as Important Molecules of Plant Interactions with the Environment", MOLECULES, vol. 19, no. 10, 10 October 2014 (2014-10-10), pages 16240-16265 reports on flavonoids in general and discloses inter alia the use of these flavonoids in the treatment of dandruff. Myricetin is mentioned in a long list of flavonoids and in addition not in the context of anti-dandruff effects. WO 03/096998 A1 describes anti-dandruff shampoos containing active substances of the polyphenol type, particularly of the catechin and/or flavonoid type. However, myricetin is not mentioned.

### Summary of the Invention

In a first aspect the invention provides a composition for topical application to the scalp or hair for use in the treatment and/or prevention of dandruff, the composition comprising, as an active anti-dandruff agent, myricetin and/or a derivative of myricetin, wherein the derivative comprises a substitution in place of one or more of the OH groups attached to the ring structure, wherein the substitution comprises a sugar, a lower alkyl or a substituted lower alkyl group.

In a second aspect the invention provides for the non-therapeutic use of myricetin and/or a derivative of myricetin, wherein the derivative comprises a substitution in place of one or more of the OH groups attached to the ring structure, wherein the substitution comprises a sugar, a lower alkyl or a substituted lower alkyl group, as an active anti-dandruff agent in a composition for topical application to the scalp and/or hair.

The text describes a method of making an anti-dandruff composition for topical application to the scalp and/or hair, the composition comprising myricetin and/or a myricetin derivative as an active anti-dandruff agent, the method comprising the steps of:
a) mixing the myricetin and/or myricetin derivative with a dermatologically acceptable diluent or carrier; and
b) packaging the resulting mixture from step (a) in suitable packaging.

In this specification, "dermatologically acceptable" means suitable for contact with human skin tissue without causing undue toxicity, allergic response, inflammation or the like.

The text describes a method of preventing and/or treating dandruff in a human subject, the method comprising the step of applying to the scalp and/or hair of the subject in efficacious amounts, myricetin and/or a myricetin derivative sufficient to prevent and/or treat dandruff.

The method of preventing and/or treating dandruff may be purely cosmetic.

For present purposes, "preventing" includes preventing or delaying the appearance of skin flakes on the scalp or in the hair of an individual. "Treating" means causing the discontinuation of skin flake formation in an individual who has dandruff at the time the composition is applied, or at least causing a detectable improvement in the condition by causing a reduction in the mean number and/or mass of skin flakes in the skin or hair of the individual.

Mean skin flake number or mass can be ascertained by, for example, brushing or combing the subject's hair for a defined number of strokes and collecting and counting the skin flakes recovered, or measuring their mass using a sensitive laboratory weighing balance.

The method of preventing and/or treating dandruff will typically require repeated application of the composition of the first aspect of the invention to the scalp and/or hair of the subject. The composition may be administered preferably at least weekly for a preventative effect, and may be used more frequently e.g. daily, especially for treatment of a subject who has active dandruff (i.e. for a treatment effect).

In particular, the method will typically comprise application to the scalp and/or hair of the subject of sufficient amounts of myricetin and/or myricetin derivative so as to achieve a local concentration on the surface of the subject's skin and/or hair which is greater than about 5µg/ml, preferably about 6µg or more. Desirably the local concentration of myricetin and/or myricetin derivative is 6.25 µg/ml or more. The upper limit of the local concentration of myricetin and/or myricetin derivative may be whatever can be achieved whilst remaining compatible with the dermatological acceptability requirement. As an illustration, the local concentration may be up to 10µg/ml, or 20µg/ml, or 40µg/ml or even up to 100µg/ml of myricetin and/or myricetin derivative.

The concentration of myricetin and/or myricetin derivative in the composition of the invention will preferably be above 5µg/ml, more preferably above 6µg/ml, and most preferably at or above 6.25µg/ml. Possibly the concentration of myricetin and/or myricetin derivative in the composition may need to be higher than this to achieve the preferred local concentrations on the subject's skin and/or hair. Equally however, it may be possible to achieve the desired local concentration on the subject's skin and/or hair using a composition with a lower concentration of myricetin and/or myricetin derivative if these substances accumulate after repeated application of the composition.

The composition conveniently takes the form of a shampoo and/or conditioner or a lotion, for topical application to the scalp and/or hair of the subject.

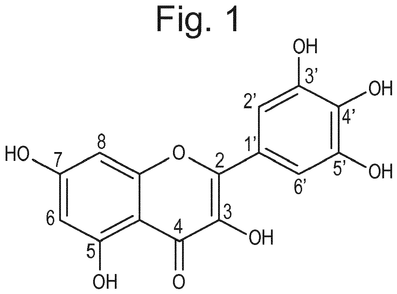

The structure of myricetin is shown in Figure 1. Myricetin is a naturally occurring compound found in fruit (especially berries), some vegetables, nuts, tea and in red wine.

For present purposes, a derivative of myricetin means a compound having the same fundamental ring structure as myricetin but comprising a substitution in place of one or more of the OH groups attached to the ring structure.

Examples of suitable substitute groups include sugars (especially hexose or pentose sugars), lower alkyl (C₁-C₄, preferably C₁ or C₂), and substituted lower alkyl groups. Conveniently the derivative is substituted at only one position. A preferred derivative comprises a substitution at the 3 position.

Desirably the derivative is a glycoside, such as a glucoside, galactoside or rutinoside. Preferably the derivative is myricetin-3-glucoside or myricetin-3-galactoside.

The myricetin and/or myricetin derivative, being an active anti-dandruff agent, is typically present in the composition at a concentration in the range 0.0001-15.0% w/w, preferably in the range 0.001-10.0% w/w, more preferably in the range 0.005-10.0% w/w, and most preferably in the range 0.01-10% w/w. A typical preferred concentration might be in the range 0.025-5.0% w/w. Where both myricetin and one or more myricetin derivatives are present in the composition, it is intended that their total combined concentration will be within the preferred ranges set out above.

Briefly, by way of explanation, the applicant has found that the pattern of gene expression, in an experimental model of human skin cells, from subjects with dandruff is different from the pattern observed in subjects without dandruff and that, surprisingly, data from the addition of myricetin to MCF7 cells (a breast cancer cell line) showed that myricetin can cause a pattern of gene expression changes which is largely the reverse of those changes seen in skin biopsies from subjects with dandruff compared to healthy subjects. Accordingly, the applicant hypothesises that myricetin, and/or derivatives of myricetin, should be effective as active agents in the prevention and/or treatment of dandruff, by reversing or counteracting the effects that dandruff causes in terms of patterns of gene expression.

### Detailed Description of the Invention

Advantageously the composition of the invention is a liquid or gel at 20°C. The myricetin and/or myricetin derivative will typically be present in the composition in solution, but may alternatively be present as a solid, a colloid, an emulsion or any other convenient form.

As noted above, the composition is typically a cosmetic composition such as a shampoo and/or conditioner, but could be any formulation which is suitable for topical application to the scalp and/or hair.

Myricetin is only sparingly soluble in water (solubility of about 55mg/L at 25°C). Accordingly, where the composition is an aqueous liquid, it may be advantageous to include a solvent for myricetin which is acceptable for topical application to the skin. Suitable solvents include alcohols, such as ethanol, isopropanol, and mixtures thereof. An alcohol or other acceptable myricetin solvent may be present in the formulation in the range 0.1-25% v/v, preferably 0.5-20% v/v, more preferably 1-10% v/v.

Where the composition comprises a myricetin derivative but essentially no myricetin, it may be preferred to avoid the use of a solvent other than water since, depending on the identity of the derivative, it may have sufficient solubility in water. For example, myricetin glycosides generally have greater solubility in water than does myricetin.

The composition may include a plurality of different myricetin derivatives, or may comprise a single myricetin derivative. The one or more myricetin derivatives may be present as the sole anti-dandruff active agent, or may be present in combination with myricetin and/or other known anti-dandruff agents. Conversely, myricetin may be present in the composition as the sole active anti-dandruff agent, or again in combination with other known anti-dandruff agents.

Preferably, in addition to myricetin and/or a myricetin derivative, the composition may comprise a conventional known anti-dandruff active agent. Such conventional additional agents include anti-microbial agents and, especially, anti-fungal agents. Preferred antifungal agents typically display a minimum inhibitory concentration of about 50 mg/ml or less against *Malassezia spp.*

Suitable additional anti-dandruff agents include one or more compounds selected from the group consisting of azole-based antifungal agents, octopirox, metal pyrithione salts, selenium sulphide compounds, and mixtures thereof. The preferred azole-based antifungal agents are ketoconazole and climbazole, but other azole compounds (e.g. clotrinazole, econazole, isoconazole and miconazole) may be suitable. Preferred metal pyrithione salts are zinc, copper, silver and zirconium pyrithione, of which the most preferred is zinc pyrithione.

Other anti-dandruff agents which may preferably be present in the composition include one or more of tripeptide 1 (i.e. the tripeptide glycine-histidine-lysine; GHK), with or without associated copper (GHK tripeptide has a high affinity for Cu II ions); melatonin - see US 6,048,886; and diprophylline (also referred to as Dyphylline) - see DE 2842472.

Preferably, any additional anti-dandruff agent or agents is/are present at from 0.01 to 10% wt. of the composition, more preferably from 0.1 to 5.0% wt. of the composition.

As well as additional conventional anti-dandruff agents, the composition of the invention may further comprise one or more conventional ingredients of shampoo and/or conditioner formulations including, but not limited to, the following: water; a cleansing surfactant; a conditioning agent; suspending agent; fragrance; dye or pigment; pH adjusting or regulating agent; pearlescer or opacifier; viscosity modifier; and preservative. Generally these optional ingredients may each be included individually up to an amount of about 5% w/w of the total composition.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di-and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, trieltanolamine dodecylbenzene sulphonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective formulae R₂OSO₃M and RiO (C₂H₄O)xS0₃M, wherein R₂ is alkyl or alkenyl of from 8 to 18 carbon atoms, x is an integer having a value of from about 1 to about 10, and M is a cation such as ammonium, alkanolamines, such as triethanolamine, monovalent metals, such as sodium and potassium, and polyvalent metal cations, such as magnesium, and calcium. Most preferably R₂ has 12 to 14 carbon atoms, in a linear rather than branched chain.

Preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=l.

Preferably the level of alkyl ether sulphate is from 0.5 wt to 25 wt% of the total composition, more preferably from 3 wt to 18 wt%, most preferably from 6 wt to 15 wt% of the total composition.

The total amount of anionic cleansing surfactant in compositions of the invention generally ranges from 0.5 wt to 45 wt%, more preferably from 1.5 wt to 20 wt%.

Compositions of the invention may contain non-ionic surfactant. Most preferably non-ionic surfactants are present in the range 0 to 5 wt%.
Nonionic surfactants that can be included in compositions of the invention include condensation products of aliphatic (C8 - C18) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula R-(OCH₂CH₂)nOH, where R is an alkyl chain of C12 to C15, and n is 5 to 9.

Other suitable nonionic surfactants include mono- or di-alkyl alkanolamides. Examples include coco mono- or di-ethanolamide and coco mono-isopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Suitable alkyl polyglycosides for use in the invention are commercially available and include for example those materials identified as: Oramix NS10 ex Seppic; Plantaren 1200 and Plantaren 2000 ex Henkel.

Amphoteric or zwitterionic surfactant can be included in an amount ranging from 0.5 wt to about 8 wt, preferably from 1 wt to 4 wt of the total composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in compositions of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.
Compositions according to the invention such as shampoos and conditioners suitably contain conditioning agents such as silicone conditioning agents and non-silicone oily conditioning agents.

Suitable silicone conditioning agents include polydiorganosiloxanes, in particular polydimethylsiloxanes which have the CTFA designation dimethicone. Also suitable for use in compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188. These materials can impart body, volume and stylability to hair, as well as good wet and dry conditioning. Also suitable are functionalised silicones, particularly amino-functionalised silicones.

Suitable non-silicone oily conditioning agents are selected from hydrocarbon oils, fatty esters and mixtures thereof.

The further conditioning agent is suitably present in shampoo or conditioner compositions at a level of from 0.05 to 10, preferably from 0.2 to 5, more preferably from about 0.5 to 3 percent by total weight of further conditioning agent based on total weight of the composition.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent, if included, will generally be present in a composition of the invention at levels of from 0.1 to 10%, preferably from 0.5 to 6%, more preferably from 0.9 to 4% by total weight of suspending agent based on the total weight of the composition.

The composition typically comprises at least 30% of water by weight of the composition, more preferably from 35 to 95%, even more preferably from 45 to 88%, still even more preferably from 55 to 82%, most preferably from 65 to 80% by weight of the total composition.

Compositions of the invention are primarily intended for topical application to at least a portion of the scalp and/or hair of an individual, either in rinse-off or leave-on compositions, for the prevention and/or treatment of dandruff.

Preferably, the composition has a viscosity of less than 200,000 centipoise. More preferably, the composition has a viscosity of no greater than 50,000 centipoise, even more preferably no greater than 20,000 centipoise, and most preferably no greater than 10,000 centipoise. It is preferred that the composition has a viscosity of at least 10 centipose. More preferably, the composition has a viscosity of at least 200 centipose, even more preferably at least 1000 centipose and still even more preferably at least 3000 centipose. The viscosity of the present invention is taken at 30°C with a Brookfield Viscometer, Spindle No. 5 at a speed of 20 rpm.

The invention will now be described by way of illustrative example and with reference to the accompanying drawing, in which:
Figure 1 shows the structural formula of myricetin;
Figure 2A is a bar chart showing the expression of various genes that are significantly upregulated in subjects with dandruff compared to healthy scalp skin - the left hand y axis shows the amount of increase in expression (log fold change);
Figure 2B is a bar chart similar to that shown in Figure 2A, but shows genes whose expression is significantly downregulated in dandruff scalp skin compared to healthy scalp skin - again the left hand y axis shows the amount of change in expression (log fold change) which, in Figure 2B, is negative; and
Figure 3 is a bar chart showing the amount of IL-8 (pg IL8 per 100µg protein) released from cultured primary human keratinocytes in negative and positive control cultures and in the presence of various concentrations of myricetin, following stimulation with a cocktail of inflammatory cytokines (IL-17, IL-22, TNFα). Data from the controls were used in a statistical comparison with those from the myricetin treatments using Student's t-test.

### Examples

### Example 1

It is fundamentally challenging to identify novel intervention routes for diseases and cosmetic conditions. Approaches are needed to try and match the action of small-molecule therapeutics with diseases and physiological processes. One approach that has been developed to address this is The Connectivity Map developed by the Broad Institute of MIT (Lamb et al., Science 2006 313, 1929-1935). The goal of this project is to provide a generic solution to this problem by attempting to describe all biological states in terms of a genomic signature, create a large public database of signatures and genes and develop pattern-matching tools to detect similarities among these signatures. The approach uses perturbations in mammalian cell culture models which will provide an approach that is generalised, systematic and biologically relevant. To generate the small molecule gene expression signatures, high throughput treatments of a breast cancer epithelial cell line, MCF7, a prostate cancer cell line PC3, and the non-epithelial leukaemia cell line HL60 at various doses were completed. Reasonably high doses in the region of 10mM were selected and the signatures were obtained at relatively early time points of 6 or 12 hrs. The data for the first 164 molecules was collected over 1 year using Affymetrix GeneChip microarrays. The database has now grown to over 1300 perturbagens.

The Connectivity Map uses a rank-based pattern matching strategy based on the Kolmogorov-Smirnov statistic rather than the more traditional approach of hierarchical clustering normally used with this data type. The approach starts with a "query signature" and assesses its similarity to the reference expression profiles in the data set. Each gene in the signature carries a sign indicating if it is up or down regulated. The reference gene-expression profiles in the Connectivity Map are also represented in a nonparametric fashion. Each profile is compared to its corresponding intra-batch vehicle-treated control. The genes on the array are rank-ordered according to their differential expression relative to the control; each treatment instance thus gives rise to a rank-ordered list of -22,000 genes. The query signature is then compared to each rank-ordered list to determine whether up-regulatory query genes tend to appear near the top of the list and down-regulated query genes near the bottom ("positive connectivity") or vice versa ("negative connectivity") yielding a connectivity score ranging from +1 to -1. All instances in the database are ranked according to their connectivity scores; those at the top are most strongly correlated to the query signature and those at the bottom are most strongly anti-correlated.

Other researchers have built on the Connectivity Map suggesting refinements to the statistical analysis (Zhang 2008 BMC Bioinformatics 9, 258; & Cheng Pac. Symp. Biocomput. 2013: 5-16). A number of publications have shown the power of this approach with examples including, identifying thioridazine as an inhibitor of the PI3K/AKT pathway (Rho 2011 Gynecol. Oncol. 120, 121-127), identifying inhibitors of epithelial-mesenchymal transition (Reka 2011 J. Thorac. Oncol. 6;1784-92), showing that the antipsychotic drug trifluoperazine inhibits cancer stem cell growth (Yeh 2012 Am J. Respir. Crit. Care Med. 186, 1180-1188), predicting novel hERG inhibitors (Babcock 2013 PLoS One. 8: e69513), repurposing the antihelmintic mebendazole for treating colon cancer (Nygren 2013 J. Cancer Res. Oncol. 139, 2133-40) and identifying thiostrepton as a novel agent to target human colon cancer stem cells (Ju et al. 2015 Cell Death Dis. 6: e1801).

Application of the The Connectivity Map is now extending beyond pharmaceuticals with some publications demonstrating its use in the cosmetic field, for example in identifying the molecular mechanisms of traditional Chinese medicine formulations (Wen et al. 2011 PLoS One 6: e18278).

The inventors generated a high quality transcriptomics data set using 4mm skin biopsies sampled from the scalps of (n=21) healthy and dandruff-afflicted subjects. The total RNA from the biopsies was extracted and analysed using the whole genome Agilent microarrays and Gene Spring software. The resulting data set was analysed using the Connectivity Map approach to identify novel putative anti-dandruff agents.

### Connectivity Map Analysis

A query signature, using the list of genes identified as significantly differentially expressed (adjusted p<0.05 and fold change >1.5) when comparing dandruff subjects relative to healthy subjects, was generated for querying the Broad Institute Connectivity Map. The query signature required by the Connectivity Map database is formed of up- and down-tag lists, representing a selection of up- and down-regulated probes respectively. Each tag list is specifically defined as a list of Affymetrix HG-U133A probes (Affymetrix GeneChip Human Genome U133A Array). The "Dandruff" versus healthy study was performed using the Agilent whole human genome oligo microarray G4851B. Therefore, differentially expressed probes from this comparison had to be converted from Agilent to Affymetrix format. This was performed by mapping the corresponding Entrez Gene IDs from the Dandruff (D1) vs Healthy signature annotation.

The Dandruff vs Healthy gene list contains 556 probes including 290 up- and 266 down-regulated genes; after conversion from Agilent to Affymetrix HG-U133A probe identifiers, 230 up- (79%) and 170 down- (64%) regulated Affymetrix probes were identified and formed the query signature (up-tag list and down-tag list).

Datasets from treatment with 1309 perturbagens (drugs or non-drug bioactive compounds) with a total of 6100 instances (i.e. one treatment and vehicle pair) were generated via the Broad Institute Connectivity Map and were probed with the Dandruff gene signature using the Connectivity Map algorithm.

### Connectivity Mapping Results

Following a filtering process based on the mean of the connectivity score and the number of instances, 21 perturbagens were identified. They represent the best negatively correlated perturbagens with the dandruff vs healthy gene list. Myricetin was within this 'hit list' of perturbagens:

| **Perturbagen** | **Connectivity Score** | **Cell Types and Concentration** | **Chemical Class** |
|---|---|---|---|
| Myricetin | -0.46 | HL60 13 uM | Flavonoid |
| | | MCF7 13 uM | |
| | | PC3 13 uM | |

Myricetin is a naturally-occurring substance and is already a recognised cosmetic ingredient. It is therefore a good choice for inclusion in an anti-dandruff treatment formulation.

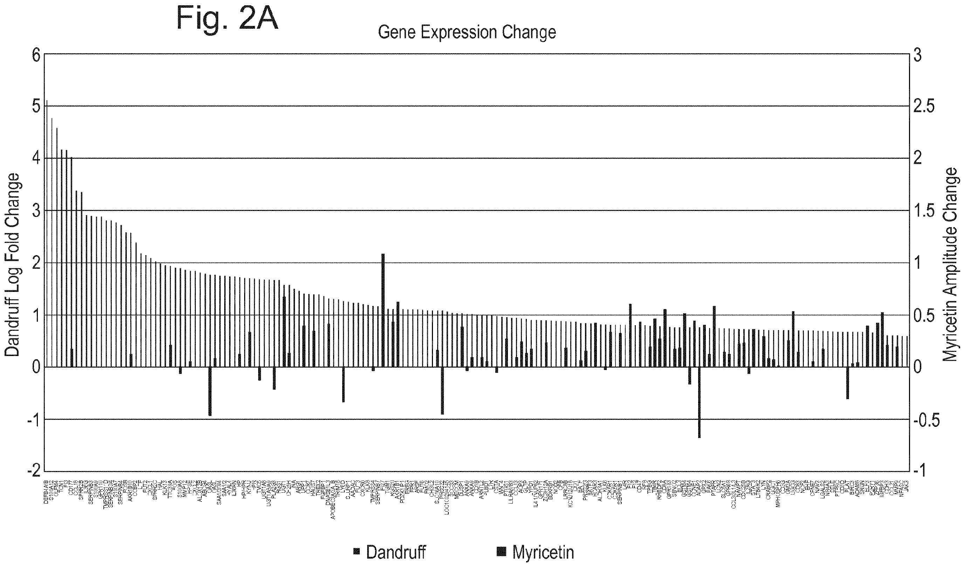

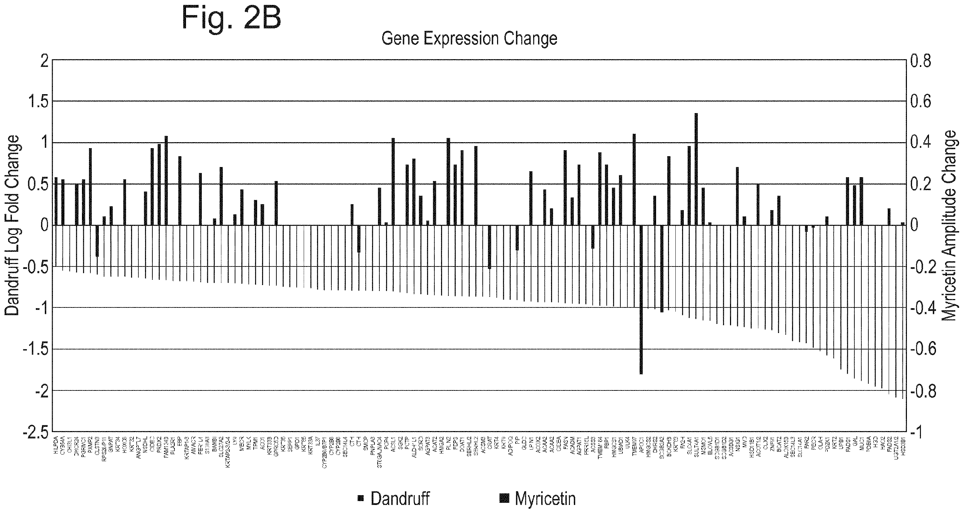

Figures 2A and 2B illustrate the complementarity of the myricetin-induced gene expression changes in the c-map data-base against the gene expression changes seen in dandruff. In particular many of the genes down-regulated in dandruff are up-regulated by myricetin treatment *in vitro.*

In Figure 2A, the light coloured bars show the changes in expression of genes that are significantly upregulated in dandruff scalp skin compared to healthy scalp skin, and the amount of change (Log fold change) is represented on the left hand y axis. The dark bars show if the expression of these same genes was altered in cultured MCF7 cells treated with 13µM myricetin compared to a DMSO vehicle control. The change in gene expression in this *in vitro* experiment was measured in amplitude and is shown on the secondary y axis (right hand side). Each gene is represented by 1 bar on the x axis.

Figure 2B is similar to Figure 2A, but in this instance the chart shows the genes whose expression is significantly downregulated in dandruff scalp skin compared to healthy scalp skin and what effect myricetin had (dark bars) on the expression of the same genes in *in vitro* culture.

These results show that myricetin treatment can reverse some of the gene expression changes which are observed in dandruff scalp skin relative to healthy scalp skin - where genes are more highly expressed in dandruff skin myricetin can, in many instances reduce the amount of increase (dark bars in Figure 2A) and in some cases actually cause a decrease in expression of those genes. Conversely, where the expression of various genes is inhibited in dandruff skin relative to healthy scalp skin (Figure 2B), myricetin can, in many instances, actually cause an increase in expression (dark bars). Thus, myricetin should be able to restore a pattern of gene expression in dandruff subjects which more nearly resembles that found in healthy subjects and may prevent and/or ameliorate the condition.

### Example 2

To test the foregoing hypothesis, the inventors devised an *in vitro* model for the inflammatory conditions prevalent in the skin of subjects with dandruff. The model involves *in vitro* tissue culture of normal human epidermal keratinocytes (HEKa cells). The cells were exposed to a combination of IL-17, TNFα and IL-22 to stimulate an inflammatory-type response, which response was measured by production of IL-8 by the keratinocytes and also by analysis of their general gene expression profile.

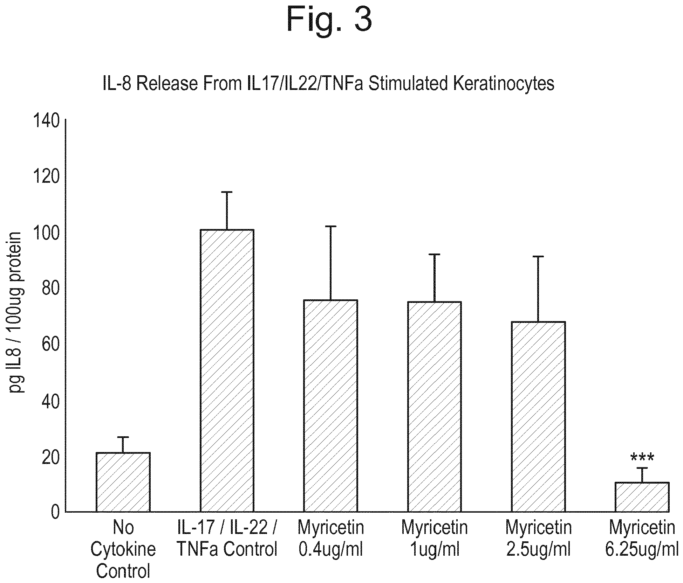

In this model, the addition of myricetin to the keratinocyte culture inhibited the inflammatory response, as indicated by a significant reduction in the production of IL-8 (see Figure 3). A number of other potential agents were tested in the model without showing any significant effect on levels of IL-8 production.

### Protocol & Materials

### Cells

Adult proliferating human epidermal karatinocytes (HEKa) (from Thermo Fisher Scientific) used at low passage (p3 or lower).

### Media

Cells were cultured in keratinocyte maintenance medium KGM Gold (Lonza Group) containing all components of the bullet kit plus 70µM calcium. For the assay, cells were plated in KGM Gold with all components of the bullet kit except hydrocortisone and gentamycin, supplemented with 70µM calcium.

### Assay

24 well tissue culture plates were seeded with 20,000 keratinocyte cells per well in plating medium and allowed to settle and spread for 24 hours at 37°C in 5% CO₂ in a cell culture incubator. The plating medium was then removed and replaced with test medium for 16hrs. The test medium contained the recombinant cytokine protein cocktail IL-17 at 200ng/ml, IL-22 at 200ng/ml & TNFa at 10ng/ml (Sigma Aldrich UK) and the test active of interest, myricetin (Sigma Aldrich), re-suspended in dimethyl sulfoxide (DMSO) applied at doses ranging from 0.4µg/ml-6.5µg/ml. A negative control in which the cytokines and equivalent amount of DMSO was applied but omitting the myricetin was also included. After 16hrs the cell culture medium was removed and the amount of IL-8 cytokine released from the keratinocyte cells was quantified using a DuoSet IL-8 ELISA kit (R&D Systems Ltd). The keratinocyte cells were lysed in RIPA buffer and the total amount of protein in each sample quantified using a Pierce BCA protein assay kit (Thermofisher Ltd). This protein data was used to normalise the IL-8 cytokine data. All treatments were run in triplicate and a mean and standard deviation value calculated, the results being illustrated in Figure 3. It can be seen that the presence of myricetin, especially at a concentration of 65µg/ml, caused a marked inhibition of the inflammatory response to the cytokines. This result was statistically significant (p = 0.0001) when analysed using one way ANOVA followed by paired Student's t-test.

### Example 3

This example relates to exemplary embodiments of compositions in accordance with, and/or for use in the method of, the invention.

### Shampoo Formulations

| **Ingredient % wt.** | **Shampoo 1** | **Shampoo 2** |
|---|---|---|
| Sodium Laureth Sulphate | 16 | 14 |
| Cocoamidopropyl betaine | 2 | 1.6 |
| Zinc Pyrithione | -- | 1.0 |
| Zinc Sulfate | -- | 0.1 |
| Silicone | 3.0 | 2.0 |
| PEG viscosifer | 2.0 | 2.0 |
| Acrylic Acid Polymer (Carbomer) | -- | 0.6 |
| Ethyleneglycol Distearate (EGDS) | 1.5 | -- |
| Guar Hydroxypropyl Trimonium Chloride | 0.1 | 0.2 |
| Salt, Preservatives and Perfumes | 1.0 | 1.5 |
| Isopropanol | 2.5 | 2.5 |
| Myricetin | 1.0 | 1.0 |
| Myricetin-3-glucoside | -- | 0.5 |
| Climbazole | 1.0 | -- |
| Water | to 100 | to 100 |

### Sample Hair/Scalp Lotion Formulations

| **Component % wt.** | **Lotion 1** | **Lotion 2** |
|---|---|---|
| Myricetin | 1.0 | 2.5 |
| Acrylates/C 10-30 Alkyl Acrylate Crosspolymer | 0.40 | 0.40 |
| Sodium Hydroxide | 0.10 | 0.10 |
| Preservative | 0.11 | 0.11 |

| **Component % wt.** | **Lotion 1** | **Lotion 2** |
|---|---|---|
| Disodium EDTA | 0.05 | 0.05 |
| Polyoxyethylene 7 lauryl alcohol | 1.00 | 1.00 |
| PPG-1-PEG-9 Lauryl Glycol Ether | 3.00 | 4.00 |
| Decamethylcyclopentasiloxane | 0.48 | 0.48 |
| Dimethicone | 0.04 | 0.04 |
| Fragrance | 0.5 | 0.5 |
| Ethanol | 5.0 | 6.0 |
| Zinc pyrithione | 0.5 | -- |
| Diprophylline | 0.5 | 0.5 |
| Water | to 100 | to 100 |

## Claims

1. Non-therapeutic use of myricetin and/or a derivative of myricetin, wherein the derivative comprises a substitution in place of one or more of the OH groups attached to the ring structure, wherein the substitution comprises a sugar, a lower alkyl or a substituted lower alkyl group, as an active anti-dandruff agent in a composition for topical application to the scalp and/or hair.

2. The use according to claim 1, wherein the composition is a shampoo and/or a conditioner.

3. The use according to claim 1 or 2, wherein the composition comprises one or more additional anti-dandruff agents.

4. The use according to claim 3, wherein the one or more additional anti-dandruff agents are selected from the group consisting of: ketoconazole; climbazole; metal pyrithione salts, preferably zine, copper, silver or zirconium pyrithione; diprophylline; and tripeptide GHK.

5. The use according to any one of the preceding claims, wherein the composition is a shampoo and/or conditioner and further comprises any combination, or all, of the following: water; a cleansing surfactant; a conditioning agent; suspending agent; fragrance; dye or pigment; pH adjusting or regulating agent; pearlescer or opacifier, viscosity modifier; and preservative.

6. The use according to any one of the preceding claims, wherein the myricetin and/or myricetin derivative is present in the composition at a concentration in the range 0.001-10.0% w/w, preferably 0.005-10.0% w/w.

7. The use according to claim 6, wherein the myricetin and a myricetin derivative is present in the composition at a concentration in the range 0.01-10.0% w/w, preferably 0.025-5.0% w/w.

8. The use according to any one of the preceding claims, wherein the myricetin derivative comprises myricetin substituted at a single position.

9. The use according to any one of the preceding claims, wherein the myricetin derivative comprises myricetin substituted at the 3 position.

10. The use according to any one of the preceding claims, wherein the myricetin derivative is a myricetin glycoside.

11. The use according to any one of the preceding claims, wherein the myricetin derivative is myricetin-3-glucoside or myricetin-3-galactoside.

12. A composition for topical application to the scalp or hair of a human subject for use in the treatment and/or prevention of dandruff, the composition comprising myricetin and/or a derivative of myricetin, wherein the derivative comprises a substitution in place of one or more of the OH groups attached to the ring structure, wherein the substitution comprises a sugar, a lower alkyl or a substituted lower alkyl group, as an active anti-dandruff agent.

## Patentansprüche

1. Nichttherapeutische Verwendung von Myricetin und/oder einem Derivat von Myricetin, wobei das Derivat eine Substitution anstelle einer oder mehrerer der an die Ringstruktur gebundenen OH-Gruppen umfasst, wobei die Substitution einen Zucker, ein Niederalkyl oder eine substituierte Niederalkylgruppe umfasst, als Antischuppen-Wirkstoff in einer Zusammensetzung zur topischen Anwendung auf die Kopfhaut und/oder das Haar.

2. Verwendung nach Anspruch 1, wobei die Zusammensetzung ein Shampoo und/oder ein Conditioner ist.

3. Verwendung nach Anspruch 1 oder 2, wobei die Zusammensetzung ein oder mehrere zusätzliche Antischuppenmittel umfasst.

4. Verwendung nach Anspruch 3, wobei das eine oder die mehreren zusätzlichen Antischuppenmittel ausgewählt sind aus der Gruppe bestehend aus: Ketoconazol; Climbazol; Metallpyrithionsalzen, bevorzugt Zink-, Kupfer-, Silber- oder Zirkoniumpyrithion; Diprophyllin; und Tripeptid GHK.

5. Verwendung nach irgendeinem der vorhergehenden Ansprüche, wobei die Zusammensetzung ein Shampoo und/oder Conditioner ist und ferner eine beliebige Kombination oder alle der Folgenden umfasst: Wasser; ein Reinigungstensid; ein Konditionierungsmittel; Suspendiermittel; Duftstoff; Farbstoff oder Pigment; pH-Einstell- oder Regulierungsmittel; Perlglanzmittel oder Trübungsmittel, Viskositätsmodifikator; und Konservierungsmittel.

6. Verwendung nach irgendeinem der vorhergehenden Ansprüche, wobei das Myricetin und/oder Myricetin-Derivat in der Zusammensetzung in einer Konzentration im Bereich von 0,001 - 10,0 % Gew./Gew., bevorzugt 0,005 - 10,0 % Gew./Gew., vorhanden ist.

7. Verwendung nach Anspruch 6, wobei das Myricetin und/oder ein Myricetin-Derivat in der Zusammensetzung in einer Konzentration im Bereich von 0,01 - 10,0 % Gew./Gew., bevorzugt 0,025 - 5,0 % Gew./Gew., vorhanden ist.

8. Verwendung nach irgendeinem der vorhergehenden Ansprüche, wobei das Myricetin-Derivat Myricetin umfasst, das an einer einzelnen Position substituiert ist.

9. Verwendung nach irgendeinem der vorhergehenden Ansprüche, wobei das Myricetin-Derivat Myricetin umfasst, das an der 3-Position substituiert ist.

10. Verwendung nach irgendeinem der vorhergehenden Ansprüche, wobei das Myricetin-Derivat ein Myricetin-Glycosid ist.

11. Verwendung nach irgendeinem der vorhergehenden Ansprüche, wobei das Myricetin-Derivat Myricetin-3-glucosid oder Myricetin-3-galactosid ist.

12. Zusammensetzung zur topischen Anwendung auf der Kopfhaut oder dem Haar eines Menschen zur Verwendung bei der Behandlung und/oder Vorbeugung von Schuppen, wobei die Zusammensetzung Myricetin und/oder ein Derivat von Myricetin, wobei das Derivat eine Substitution anstelle einer oder mehrerer der an die Ringstruktur gebundenen OH-Gruppen umfasst, wobei die Substitution einen Zucker, ein Niederalkyl oder eine substituierte Niederalkylgruppe umfasst, als ein Antischuppen-Wirkstoff umfasst.

## Revendications

1. Utilisation non-thérapeutique de myricétine et/ou d'un dérivé de myricétine, dans laquelle le dérivé comprend une substitution en place d'un ou plusieurs des groupes OH fixés à la structure de noyau, dans laquelle la substitution comprend un sucre, un groupe alkyle inférieur ou un groupe alkyle inférieur substitué, comme un agent antipelliculaire actif dans une composition pour une application topique au cuir chevelu et/ou aux cheveux.

2. Utilisation selon la revendication 1, dans laquelle la composition est un shampoing et/ou un après-shampoing.

3. Utilisation selon la revendication 1 ou 2, dans laquelle la composition comprend un ou plusieurs agents antipelliculaires supplémentaires.

4. Utilisation selon la revendication 3, dans laquelle les un ou plusieurs agents antipelliculaires supplémentaires sont choisis dans le groupe consistant en : kétoconazole ; climbazole ; sels de pyrithione de métaux, de préférence pyrithione de zinc, cuivre, argent ou zirconium ; diprophylline ; et tripeptide GHK.

5. Utilisation selon l'une quelconque des revendications précédentes, dans laquelle la composition est un shampoing et/ou un après-shampoing et comprend de plus toute combinaison, ou la totalité, des suivants : eau ; un tensioactif nettoyant ; un agent conditionnant ; agent de mise en suspension ; parfum ; colorant ou pigment ; agent d'ajustement ou de régulation du pH ; agent nacrant ou opacifiant ; modificateur de viscosité ; et conservateur.

6. Utilisation selon l'une quelconque des revendications précédentes, dans laquelle la myricétine et/ou le dérivé de myricétine est présent dans la composition à une concentration dans l'intervalle de 0,001-10,0 % masse/masse, de préférence 0,005-10,0 % masse/masse.

7. Utilisation selon la revendication 6, dans laquelle la myricétine et un dérivé de myricétine est présent dans la composition à une concentration dans l'intervalle de 0,01-10,0 % masse/masse, de préférence 0,025-5,0 % masse/masse.

8. Utilisation selon l'une quelconque des revendications précédentes, dans laquelle le dérivé de myricétine comprend de la myricétine substituée à une seule position.

9. Utilisation selon l'une quelconque des revendications précédentes, dans laquelle le dérivé de myricétine comprend de la myricétine substituée à la position 3.

10. Utilisation selon l'une quelconque des revendications précédentes, dans laquelle le dérivé de myricétine est un myricétine glycoside.

11. Utilisation selon l'une quelconque des revendications précédentes, dans laquelle le dérivé de myricétine est le myricétine-3-glucoside ou myricétine-3-galactoside.

12. Composition pour application topique au cuir chevelu ou cheveux d'un être humain sujet à une utilisation dans le traitement et/ou la prévention de pellicules, la composition comprenant de la myricétine et/ou un dérivé de myricétine, dans laquelle le dérivé comprend une substitution en place d'un ou plusieurs des groupes OH fixés à la structure de noyau, dans laquelle la substitution comprend un sucre, un groupe alkyle inférieur ou un groupe alkyle inférieur substitué, comme un agent antipelliculaire actif.
